# EUROPEAN PATENT APPLICATION

(11) **EP 4 066 724 A1**
(43) Date of publication of application: **05.10.2022**
(21) Application number: 21315059.2
(22) Date of filing: 31.03.2021
(51) Int. Cl.: A61B 1/018, A61B 17/00, A61B 1/012, A61B 1/313, A61B 1/05, A61B 1/273, A61B 1/303, A61B 1/307, A61B 1/31

(54) **INSERTION INSTRUMENTS AND SYSTEMS**

(71) Applicant: Caranx Medical SAS, 75003 Paris (FR)
(72) Inventor: SEJOR, Eric, 06000 Nice (FR); POULETTY, Philippe, 75005 Paris (FR); BERTHET-RAYNE, Pierre, 06800 Cagnes-sur-Mer (FR)
(74) Representative: Hepp Wenger Ryffel AG

(57) **Abstract**

The invention relates to insertion instruments (50) for insertion into a cavity, the instrument (50) comprising a flexible tubular sheet (1) having a first annular end (12), a second annular end (13) and a middle section (14) extending between the first annular end (12) and the second annular end (13), a first holding member (24) holding the first annular end (12), wherein an outer tubular outer portion (16) of the middle section (14) extends along an extension direction (D), from the first holding member (24) to a distal tip (17) of the instrument (50), a second holding member (2) holding a tubular inner portion (18) of the middle section (14), wherein the inner portion (18) extends inside the outer tubular outer portion (16) of the sheet (1), from the second holding member (2) to the distal tip (17), the inner portion (18) transiting into the outer portion (16) at the distal tip (17), a passage (19) defined inside the inner tubular inner portion (18) of the sheet (1), which passage (19) extends from the second holding member (2) and opens out at the distal tip (17). In one aspect, the second holding member (2) is designed and arranged for controlling the length of the outer portion (16) of the sheet (1) along the extension direction (D) by moving the inner portion (18) of the sheet (1).

## Description

The present application relates to insertion instruments, in particular surgical insertion instruments, for insertion into a cavity, in particular in a human subject, as well as to systems comprising such instruments.

So called "growing robots" have been known for various fields of applications. Such a robot is known from US 2019/0217908 A1 which discloses an everted tubular sheet whose inner portion is wound up on a reel. By unwinding the tubular sheet from the reel and blowing a gas into a chamber enclosed by the sheet, the length of an outer portion of the sheet can be increased. However, when the sheet has been introduced into a cavity, the pressure inside the chamber usually prevents the insertion of any other device through the robot and into the cavity. Instead, in order to avoid piercing the sheet, the growing robot must be fully deployed or depressurized to allow the insertion of another device through a passage formed in the growing robot. Moreover, the instrument does not allow the removal of gases or liquids from the cavity.

US 2003/0168068 A1 discloses an instrument which can be used for medical purposes. The instrument is based on an everted tubular sheet. In one embodiment, an inflatable tubular sheet is arranged in such a way that an endoscope can be entered through a passage formed inside an inner portion of the sheet. However, since the inner portion is only loosely wrapped up in an initial state, the extension cannot be precisely controlled. Moreover, the process cannot be reversed so that the instrument cannot be easily retracted from the cavity.

Moreover, US 5,236,423 discloses a tubular sheet whose inner portion is gathered in an initial state. This sheet has the same disadvantages, i. e. the extension cannot be precisely controlled, and the process cannot be reversed so that the instrument cannot be easily retracted from the cavity.

It is an object of the present application to provide an instrument in which at least some of the disadvantages mentioned above are overcome. In particular, it should allow a controlled release of the tubular sheet and the insertion of a device such as a surgical, therapeutic or diagnostic instrument or a sensor and simplify the retraction of the instrument.

In a first aspect of the invention, this is achieved by an insertion instrument, in particular a surgical insertion instrument, for insertion into a cavity, in particular a cavity of a human subject. The instrument comprises
- a flexible tubular sheet having a first annular end, a second annular end and a middle section extending between the first annular end and the second annular end,
- a first holding member holding the first annular end, wherein an outer tubular portion of the middle section extends along an extension direction, from the first holding member to a distal tip of the instrument,
- a second holding member holding a tubular inner portion of the middle section, wherein the inner portion extends inside the outer tubular portion of the sheet, from the second holding member to the distal tip, the inner portion transiting into the outer portion at the distal tip,
- a passage defined inside the inner tubular portion of the sheet, which passage extends from the second holding member and opens out at the distal tip.

As already mentioned, the insertion instrument may be a surgical insertion instrument that can be inserted into a cavity of a human subject. For example, it may be used to explore and perform procedures on the upper gastrointestinal tract, in particular esophagus, stomach, and/or small intestine, the lower gastrointestinal tract, in particular colon and/or rectum, the biliopan-creatic tract, in particular common bile duct, intrahepatic bile ducts, cystic duct, and/or Wirsung canal, the urinary tract, in particular calyces of the kidney, ureter, bladder, and/or urethra, the respiratory tract, in particular trachea, bronchi, and/or bronchioles, the uterine cavity, the Fallopian tubes, the nasal cavities, the maxillary sinuses, the frontal sinus, the Eustachian tubes, the tear ducts, the brain ventricles, the central canal of the spina medulla, the mesencephalon aqueduct and the vessels.

However, the invention also encompasses insertion instruments which are adapted and can be used for non-medical purposes, such as for insertion into tubular structures of any kind, e. g. industrial tubes whose interiors have to be inspected, for example with the aid of a camera that can be introduced through the passage of the insertion instrument.

The distal tip is arranged in an area where the tubular sheet is everted. As will become clear from the exemplary embodiments, the inner portion and the outer portion hence dynamically change during the process of inserting the instrument. Moreover, the distal tip is not fixed but changes its location during use of the instrument. Hereinafter, the outer tubular portion and the inner tubular portion will sometimes together be referred to as the extension of the instrument. As will also become clear from the exemplary embodiments, the tubular sheet may be bent actively or passively during the process. In a bent state, the extension direction is to be understood as a local direction which changes along the extension.

A chamber may be formed which is at least partially enclosed by the inner portion of the sheet and the outer portion of the sheet. A fluid, which may be a gas or a liquid, can be introduced into the chamber in order to extend the device. When the sheet is at least essentially fluid-tight, controlling a fluid pressure inside the chamber can press the outer portion of the sheet against an interior wall of a cavity into which the instrument is inserted. Additionally, the length of the extension can be controlled at least in part by the fluid pressure and the amount of fluid inside the chamber.

As will be further explained, an interior device may be inserted through the passage defined inside the inner tubular portion, in particular a surgical, therapeutic or diagnostic instrument, a sensor, a robot (in particular an autonomous robot or a magnetically guided robot), a camera or a drug delivery system. The drug delivery system may be suitable for delivering drugs, for example encapsulated drugs.

In accordance with a first aspect of the invention, the length of the outer portion of the sheet along the extension direction can be controlled by moving the inner portion of the sheet by means of the second holding member. For example, the motion of the inner portion may be controlled via driving and/or via braking the second holding member. Preferably, the inner portion of the sheet is actively moved.

The second holding member is preferably designed and arranged to hold open the passage, in particular also in a bent state of the extension. This allows the insertion of an interior device, in particular a surgical, therapeutic or diagnostic instrument, a sensor, a robot, a camera or a drug delivery system.

The diameter of the passage may be chosen in accordance with the specific application of the device. For instance, suitable diameters for endovascular applications may lie in the range from 2 mm to 5 mm, while they may lie in the range from 5 mm to 25 mm for endoscopic applications.

Preferably, the second holding member comprises a winding device for winding up the inner portion of the sheet. This arrangement provides a small overall size of the second holding member.

The inner portion may be evertingly woundable about itself by the winding device. Therefore, when wound, a radial inner surface of the inner portion lies against a radial outer surface of the inner portion.

The winding device may comprise at least one driving wheel which is rotatable about a driving axis that is arranged transversely (i. e. at a non-zero angle) with respect to the extension direction, in particular perpendicular to the extension direction. The driving wheel may be mounted on a ball bearing or bushing and may be motorized by a motor. Brushless DC motors are especially suitable as they avoid noise and dust emission. The driving wheel and/or the motor may be forced against an inner or an outer surface of the inner portion of the sheet by at least one spring to ensure an even pressure as the thickness of the wound-up portion of the sheet changes.

The winding device preferably comprises at least two such driving wheels, wherein different circumferential portions of the inner portion of the sheet are in contact with respective driving wheels. This allows a more even winding up.

With further preference, the winding device also contains at least one positioning device which is designed and arranged such that the inner portion of the sheet is held in position between the at least one driving wheel and the positioning device. The positioning device may comprise at least one positioning wheel which is designed and arranged such that the inner portion of the sheet is held in position between the at least one driving wheel and the at least one positioning wheel. The positioning device, in particular the positioning wheel, may also be biased against the sheet by at least one spring. Such an arrangement with a winding device with a driving wheel and optionally a positioning wheel may also be advantageous for driving other growing robot devices, e. g. not necessarily having a passage as described above.

As an alternative to winding, the inner portion of the sheet may also be folded in order to control the length of the outer portion of the sheet.

The sheet is preferably made from a material that is essentially not stretchable; otherwise the sheet would rather stretch than evolve when it is inflated. The sheet may be made of a plastic film, for example LDPE or LLDPE, or it may be constituted as a coated fabric. It may also be 3D printed with flexible resins.

In a second aspect of the invention, the instrument also comprises, as in the first aspect,
- a flexible tubular sheet having a first annular end, a second annular end and a middle section extending between the first annular end and the second annular end,
- a first holding member holding the first annular end, wherein an outer tubular portion of the middle section extends along an extension direction, from the first holding member to a distal tip of the instrument,
- a second holding member holding a tubular inner portion of the middle section, wherein the inner portion extends inside the outer tubular portion of the sheet, from the second holding member to the distal tip, the inner portion transiting into the outer portion at the distal tip,
- a passage defined inside the inner tubular portion of the sheet, which passage extends from the second holding member and opens out at the distal tip.

In particular, the instrument may be designed as explained above.

According to the second aspect of the invention, the instrument further comprises at least one axially extendable stabilizing structure arranged inside the inner portion of the sheet in such a way that the inner portion of the sheet is prevented from closing the passage. The stabilizing structure may extend to the same extent as the outer tubular structure, thereby preventing the passage from closing, which facilitates the insertion of an interior device, in particular a surgical, therapeutic or diagnostic instrument, a sensor, a robot (in particular an autonomous robot or a magnetically guided robot), a camera or a drug delivery system.

The stabilizing structure may comprise a plurality of stabilizing rings which are arranged along the extension direction and, in particular, concentrically around a center line of the extension. Alternatively, the stabilizing structure may comprise at least one helical spring which extends along the extension direction and, in particular, surrounds a centerline of the extension.

The stabilizing rings or coils of the helical spring, respectively, may be connected to one another by at least one, preferably several controlling wires extending in the extension direction. Preferably, the at least one controlling wire extends through openings provided in the stabilizing rings or coils of the helical spring, respectively. By pulling or releasing the controlling wire at a proximal end of the instrument, the length of the extension can be controlled. Moreover, when several direction wires are present, the direction of a further eversion of the sheet at the distal tip can be controlled by selectively pulling or releasing the controlling wires at the proximal end of the instrument.

The helical spring and the controlling wires may be made of a metal, as for example nitinol or steel, or a plastic material. With preference, the controlling wires are essentially non-elastic in order to prevent a backlash.

The instrument may contain more than one stabilizing structure, as for example three helical springs. Each stabilizing structures may have a respective centerline extending along the extension direction, wherein the centerlines may be arranged excentrically with respect to a centerline of the extension.

In particular, when there are several stabilizing structures, inside at least one or all stabilizing structures, a respective additional fluid-tight tubular sheet may be arranged which is closed at a distal end of the stabilizing structure. These additional sheets are distinct from the tubular sheet and arranged inside the inner portion of the latter. By selectively controlling the fluid pressures inside the tubular sheets, the direction of a further eversion of the sheet at the distal tip can be controlled.

With further preference, the distal tip comprises a tip head which is attached to the stabilizing structure and arranged outside a chamber that is at least partially enclosed by the inner portion of the sheet and the outer portion of the sheet, wherein the tip head comprises a head opening forming a distal end of the passage. The tip head may add to the stability of the instrument at its distal tip.

The tip head may further comprise at least one measuring device for measuring a property of an inspected object inside a cavity, in particular at least one camera and/or at least one ultrasound probe. Other types of sensors such as pressure sensors, temperature sensors, optical sensors, e. g. for spectroscopic purposes, or electric or magnetic sensors are conceivable. Wires can be used to supply these measuring devices with energy and/or to transmit data measured by the measuring devices. The wires can be arranged, for example, along the controlling wires mentioned above. Alternatively, the wires may be arranged inside one of several helical springs whose centerlines are arranged excentrically with respect to a centerline of the extension.

The distal tip of the instrument may comprise at least one guiding wheel for guiding the sheet at its transition from its inner portion to its outer portion, wherein the guiding wheel is arranged inside the chamber that is at least partially enclosed by the inner portion of the sheet and the outer portion of the sheet.

Furthermore, the distal tip may comprise at least two, in particular four, guiding wheels which are rotatably arranged on a guiding ring that surrounds the passage. These guiding rings can aid the eversion of the sheet at the distal tip of the instrument.

The guiding wheel may be passively driven due to the evolving movement of the sheet between the guiding wheels and the tip head. Alternatively, the at least one guiding wheel may be actively driven, which further facilitates the eversion of the sheet at the distal tip of the instrument. Electrical power supply to the guiding wheels may be achieved through wires which can be arranged, for example, along the controlling wires mentioned above or inside one of several helical springs whose centerlines are arranged excentrically with respect to a centerline of the extension.

In a further aspect, the invention also relates to a set comprising an insertion instrument as disclosed above as well as at least one interior device provided for extending through the passage of the insertion instrument. In particular, the set may be a surgical set. The interior device may be a surgical, therapeutic or diagnostic instrument, a sensor, a robot (in particular an autonomous robot or a magnetically guided robot), a camera or a drug delivery system.

The invention will now be further explained with several embodiments which are shown in the following figures. While these insertion instruments are all embodied as surgical insertion instrument, the invention also encompasses non-surgical insertion instruments. In the drawings,
- Figures 1a and b: show a first inventive embodiment of an insertion instrument in a perspective view;
- Figure 2: shows a helical spring of the first embodiment in a perspective view;
- Figure 3: shows the first embodiment in a sectional side view;
- Figure 4: shows a second inventive embodiment of an insertion instrument in a sectional side view;
- Figure 5: shows a tip head of the second embodiment in a perspective view;
- Figure 6: shows a third inventive embodiment in a straight state, in a perspective view;
- Figure 7: shows the third embodiment in a bent state, in a perspective view;
- Figure 8: shows a spring and an associated additional tubular sheet of the third embodiment;
- Figure 9: shows a stabilizing structure of a fourth inventive embodiment in a perspective view;
- Figure 10: shows a fifth inventive embodiment in a side view;
- Figures 11a and b: show an inventive embodiment of a surgical set comprising an insertion instrument and an surgical or diagnostic instrument.

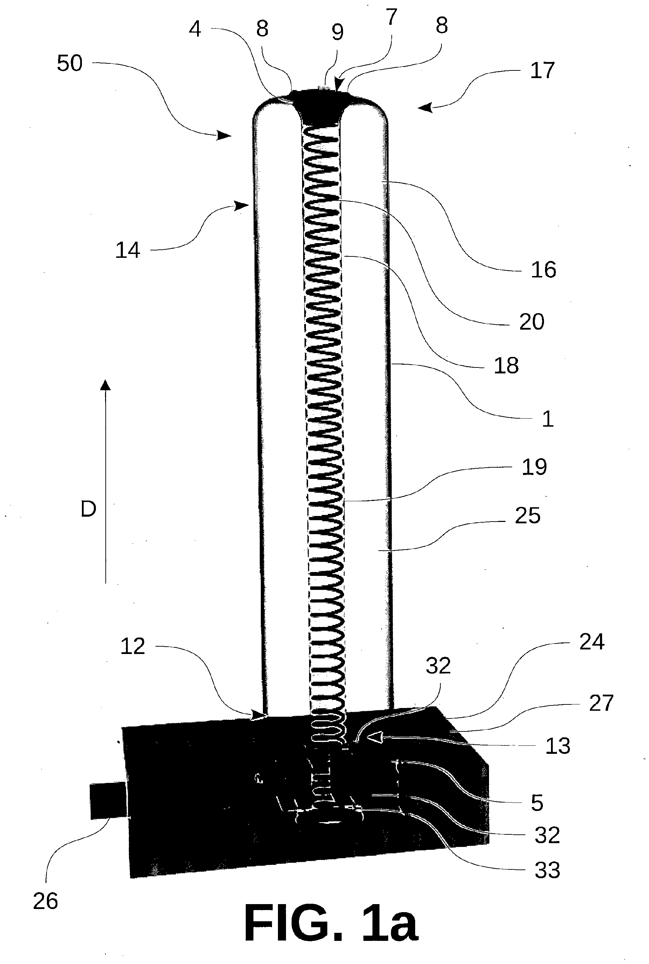

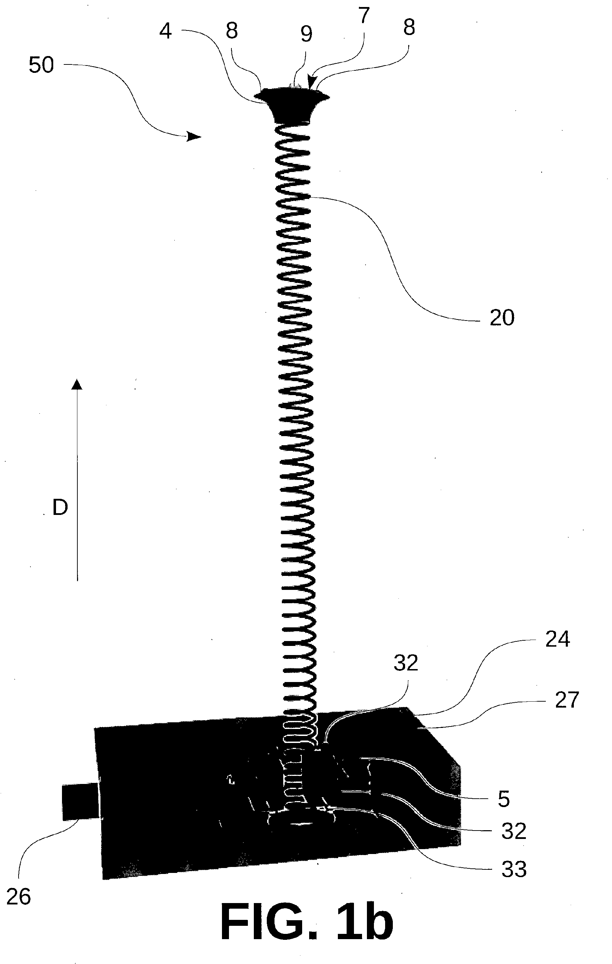

The surgical insertion instrument 50 shown in Figures 1a to 3 comprises a base member 24 and a fluid-tight flexible tubular sheet 1. The sheet 1 is only shown in Figure 1a but hidden in Figure 1b in order to more clearly display the remaining parts of the instrument 50.

The sheet 1 has a first annular end 12 that is fixed to the base member 24 which thereby forms a first holding member in terms of the present invention. The sheet 1 further comprises a second annular end 13 and a middle section 14 extending between the first annular end 2 and the second annular end 13. An outer tubular portion 16 of the middle section 14 extends along an extension direction D, from the base member 24 to a distal tip 7 of the instrument 50 where it transits into an inner tubular portion 18 arranged inside the outer tubular portion 16. The inner portion 18 and the outer portion 16 of the sheet 1 partially enclose a chamber 25 in which a fluid, in particular a gas, can be inserted. The base member 24 encloses a cavity 27 that is fluidly connected to the chamber 25. The base member 24 further contains a fluid inlet 26 through which the fluid can be entered into the cavity 27 and into the chamber 25.

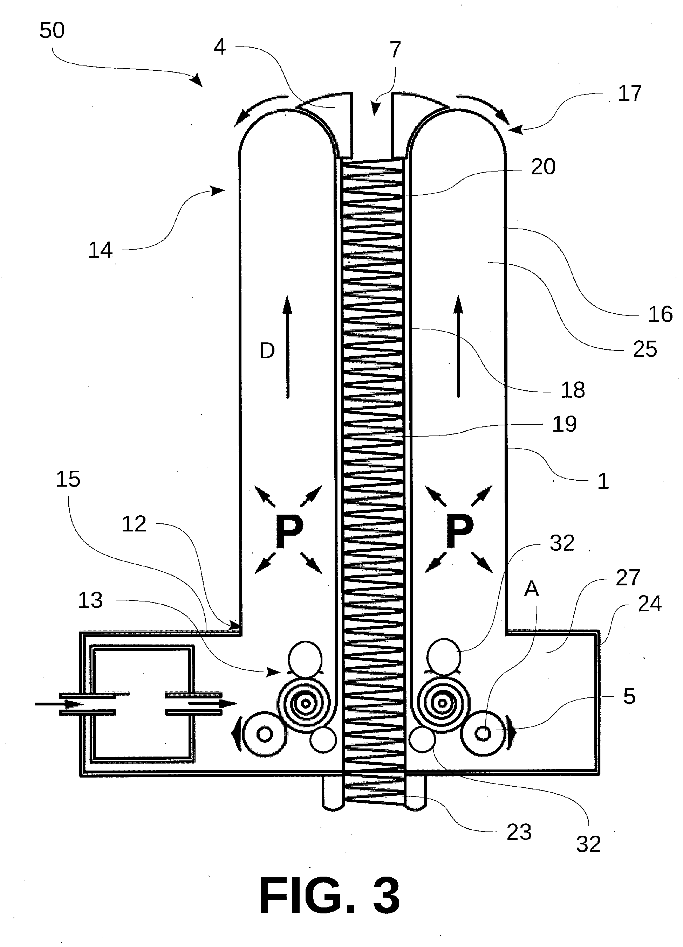

Inside the cavity 27, two driving wheels 5 are arranged which are rotatable about a respective driving axis A (see Figure 3) that is perpendicular to the extension direction D. The inner portion 18 of the sheet 1 is evertingly wound around itself, wherein different circumferential portions of the inner portion 18 of the sheet 1 are in contact with the driving wheels 5. The driving wheels 5 are motorized by motors (not shown) and can therefore control the length of the inner portion 18 of the sheet 1. The inner tubular portion 18 of the sheet 1 defines a passage 19 which extends from the driving wheels 5 and opens out at the distal tip 17. As can be recognized, the driving wheels 5 are designed and arranged to hold open the passage 19.

The winding device also contains four positioning wheels 32 which are arranged such that the inner portion 18 of the sheet 1 is held in position between the driving wheels 5 and the positioning wheels 32. The motors are forced against the inner portion 18 of the sheet 1 by springs 33 (not shown in Figure 3) to ensure an even pressure as the thickness of the wound-up portion of the sheet 1 changes.

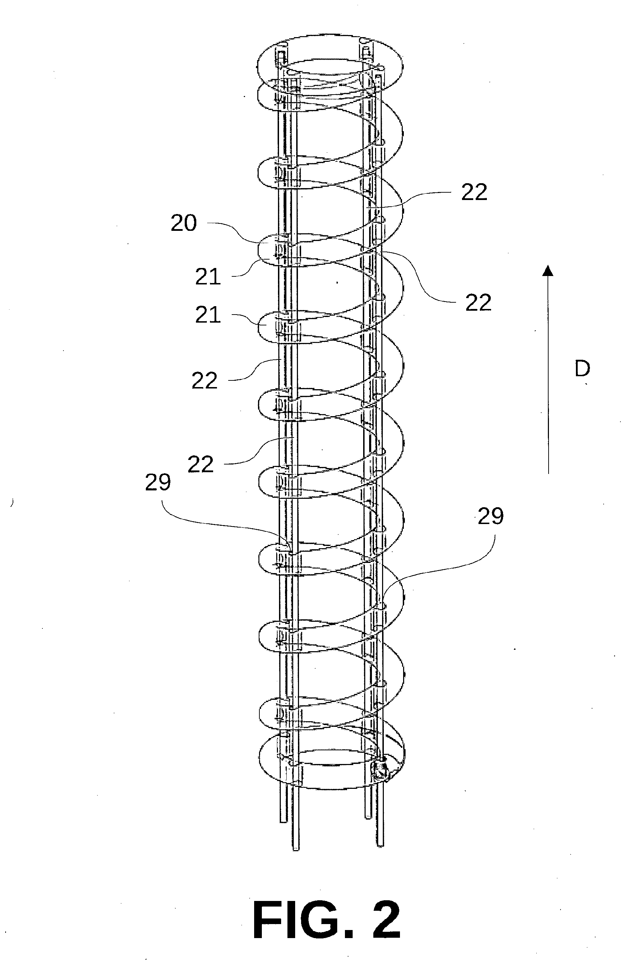

A helical spring 12 is arranged inside the inner portion 18 of the sheet 1. A proximal end 23 (see Figure 3) of the spring 20 is fixed to the base member 24 of the instrument 50. It substantially extends along the extension direction D. As best seen in Figure 2, the coils 21 of the spring 20 are loosely connected to one another by controlling wires 22. The controlling wires 22 also extend in the extension direction D and through openings 29 provided in the coils 21 of the spring 20.

The spring 20 provides a stabilizing structure which prevents the sheet 1 from closing the passage 19. This in turn allows an surgical instrument or a sensor to be inserted through the passage 19, even when the sheet 1 bends. By pulling or releasing the controlling wire 22 at a proximal end of the instrument 50, the length of the extension can be controlled. By selectively pulling the controlling wires 22 at a proximal end of the instrument 50, even the direction of further eversion of the sheet 1 at the distal tip 17 can be controlled.

The distal tip 17 of the instrument 50 comprises a tip head 4 which is attached to the spring 20 and which is arranged outside the chamber 25. The tip head 4 further comprises a head opening 7 forming a distal end of the passage 19 as well as two cameras 8 and an ultrasound probe 9. The cameras 8 and the ultrasound probe 9 may be supplied with energy through supply lines that may extend through the openings 29.

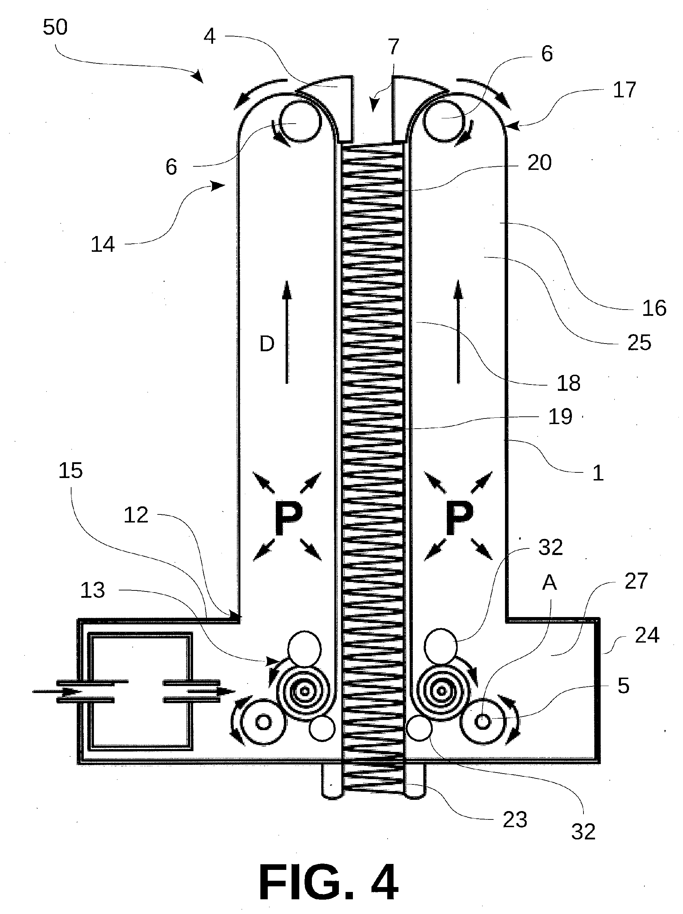

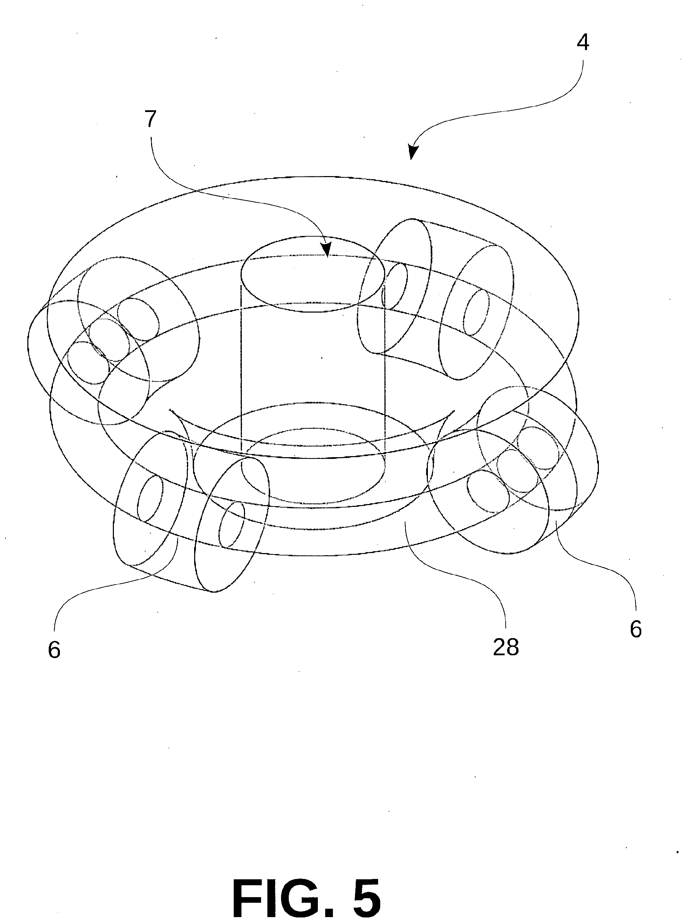

In the second embodiment shown in Figures 4 and 5, the distal tip 17 of the instrument 50 further comprise a guiding ring 28 carrying four guiding wheels 6 which are all arranged inside the chamber 25. They serve the purpose of guiding the sheet 1 (not shown in Figures 4 and 5) between the tip head 4 and the guiding wheels 6, at its transition from its inner portion 18 to its outer portion 16.

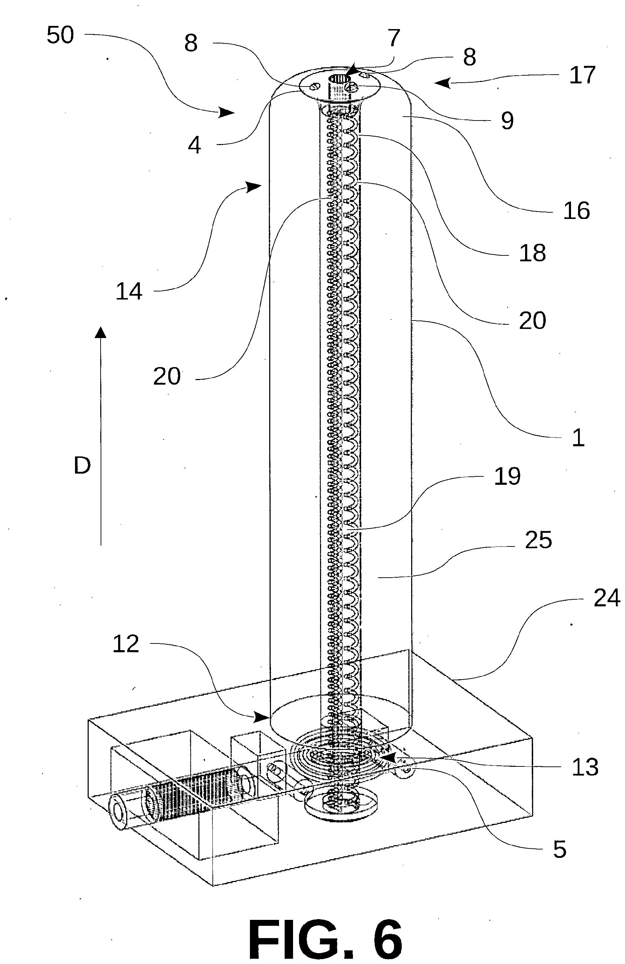

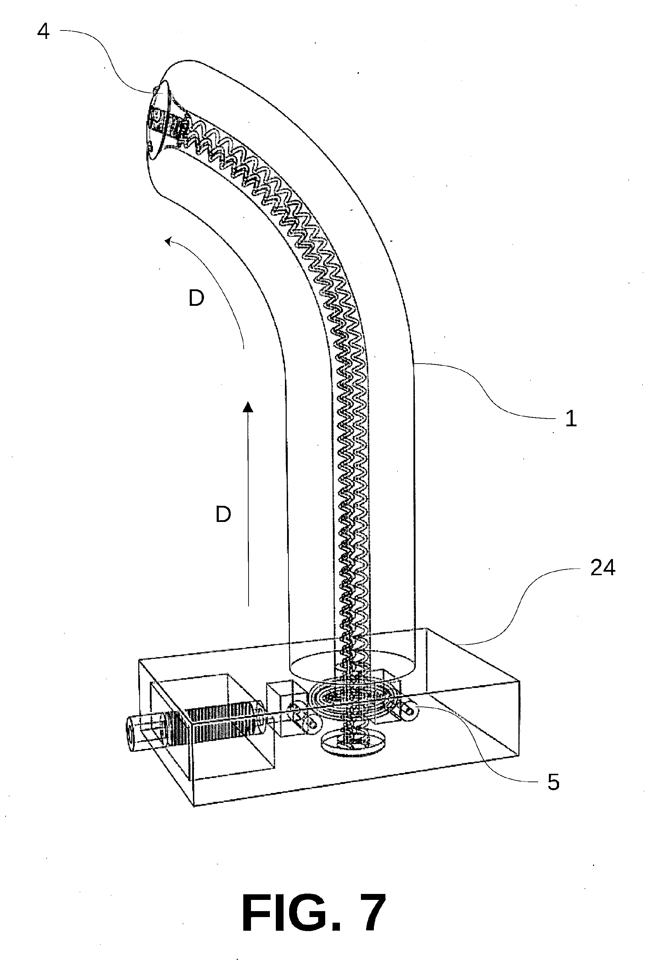

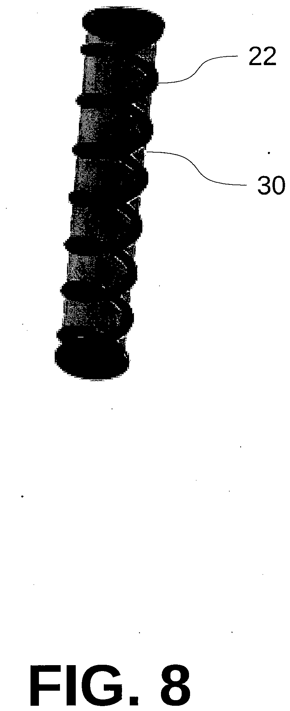

A third embodiment is displayed in Figures 6 and 7. For the purpose of simplifying the drawings, the positioning wheels and springs are not shown in Figures 6 and 7. This embodiment comprises three helical springs 20. The helical springs 20 substantially extend along the extension direction D. The coils 21 of the spring 20 are loosely connected to one another by controlling wires 22 which also extend in the extension direction D. The controlling wires 22 extend through openings 29 provided in the coils 21 of the spring 20. Proximal ends 23 of the springs 20 are fixed to the base member 24 of the instrument 50. Inside or alternatively outside each of the springs 20, a respective additional fluid-tight tubular sheet 30 is arranged which is closed at a distal end of the spring 20. A single one of the three springs 20 and its additional tubular sheet 30 are shown in Figure 8.

The coils 21 alternatively or additionally also might be connected to each other by the sheet 30.

By selectively controlling the gas pressures inside the additional sheets 30, the direction of further eversion of the sheet 1 can be controlled: Whereas Figures 6 shows a straight state of the extension, a bent state is depicted in Figure 7. In the bent state, the extension direction D changes along the extension, as indicated by the bent arrow.

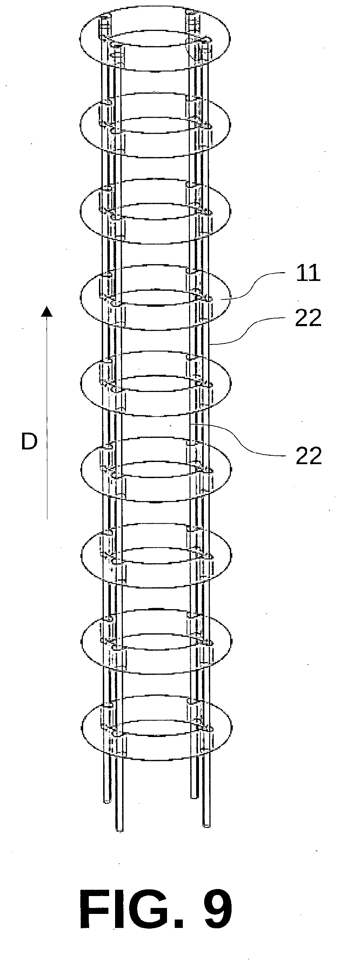

Instead of helical springs, the stabilizing structure of the fourth embodiment shown in Figure 9 contains stabilizing rings 11 which are also connected by controlling wires 22.

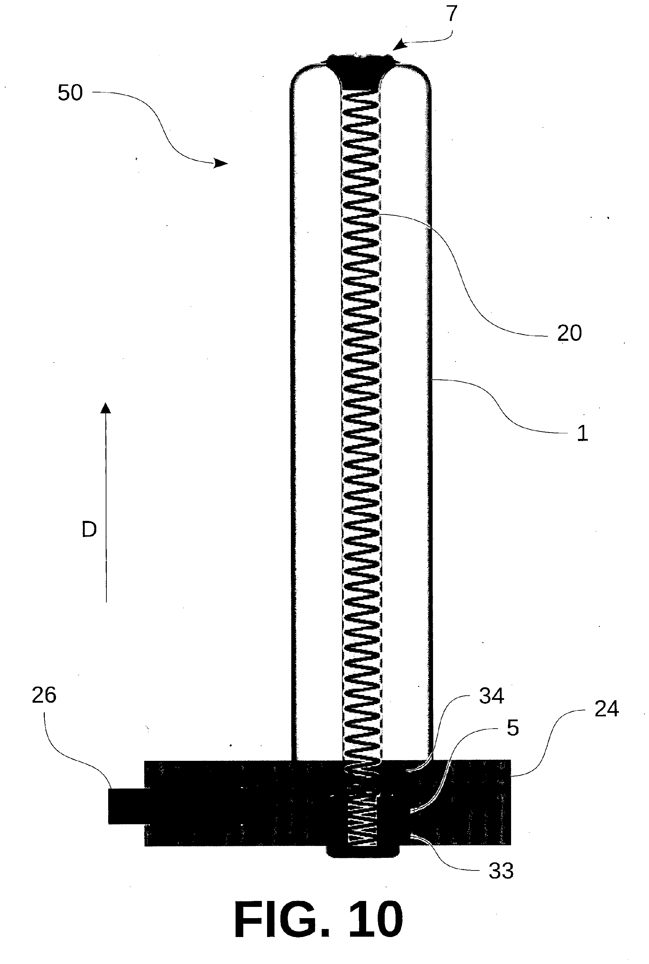

The embodiment depicted in Figure 10 does not contain any rotatable positioning wheels but fixed positioning hooks 34 which are designed and arranged such that the inner portion 18 of the sheet 1 is held in position between the driving wheels 5 and the positioning hooks 34.

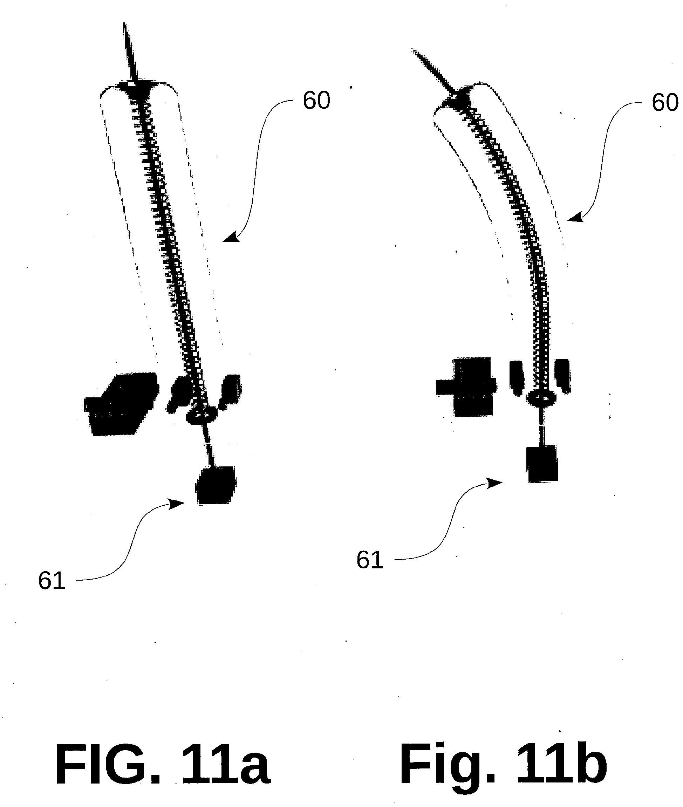

Figures 11a and 11b show a surgical set 60 in accordance with the present invention. The set 60 comprises an insertion instrument 50 as shown in Figure(s) 1 to 3 as well as a surgical instrument 61 which is provided for extending through the passage 19 of the insertion instrument 50. Again for the purpose of simplifying the drawings, the positioning wheels and springs are not shown in Figures 11a and 11b.

## Claims

1. An insertion instrument, in particular a surgical insertion instrument (50), for insertion into a cavity, the instrument (50) comprising
- a flexible tubular sheet (1) having a first annular end (12), a second annular end (13) and a middle section (14) extending between the first annular end (12) and the second annular end (13),
- a first holding member (24) holding the first annular end (12), wherein a tubular outer portion (16) of the middle section (14) extends along an extension direction (D), from the first holding member (24) to a distal tip (17) of the instrument (50),
- a second holding member (5, 32, 33, 34) holding a tubular inner portion (18) of the middle section (14), wherein the inner portion (18) extends inside the tubular outer portion (16) of the sheet (1), from the second holding member (5, 32, 33, 34) to the distal tip (17), the inner portion (18) transiting into the outer portion (16) at the distal tip (17),
- a passage (19) defined inside the tubular inner portion (18) of the sheet (1), which passage (19) extends from the second holding member (5, 32, 33, 34) and opens out at the distal tip (17),
**characterized in that**
the second holding member (5, 32, 33, 34) is designed and arranged for controlling the length of the outer portion (16) of the sheet (1) along the extension direction (D) by moving the inner portion (18) of the sheet (1).

2. The instrument (50) as claimed in claim 1,
wherein the second holding member (5, 32, 33, 34) is designed and arranged to hold open the passage (19).

3. The instrument (50) as claimed in either of claims 1 and 2, wherein the second holding member (5, 32, 33, 34) comprises a winding device (5, 32, 33, 34) for winding up the inner portion (18) of the sheet (1).

4. The instrument (50) as claimed in claim 3,
wherein the inner portion (18) is evertingly woundable about itself by the winding device (5).

5. The instrument (50) as claimed in any of the preceding claims,
wherein the winding device (5, 32, 33, 34) comprises at least one driving wheel (5) which is rotatable about a driving axis (A) that is arranged transversely with respect to the extension direction (D), wherein at least a circumferential portion of the inner portion (18) of the sheet (1) is in contact with the driving wheel (5).

6. The device as claimed in claim 5,
wherein the driving wheel (5) and/or a motor driving the driving wheel (5) is forced against the inner portion (18) of the sheet (1) by at least one spring (33).

7. The device as claimed in either of claims 5 and 6,
wherein the winding device (5, 32, 33, 34) contains a positioning device (32, 34), in particular at least one positioning wheel (32), which is arranged such that the inner portion (18) of the sheet (1) is held in position between the at least one driving wheel (5) and the positioning device (32, 34), in particular the at least one positioning wheel (32).

8. An insertion instrument, in particular a surgical insertion instrument (50), for insertion into a cavity, the instrument (50) comprising
- a flexible tubular sheet (1) having a first annular end (12), a second annular end (13) and a middle section (14) extending between the first annular end (12) and the second annular end (13),
- a first holding member (24) holding the first annular end (12), wherein a tubular outer portion (16) of the middle section (14) extends along an extension direction (D), from the first holding member (24) to a distal tip (17) of the instrument (50),
- a second holding member (2) holding a tubular inner portion (18) of the middle section (14), wherein the inner portion (18) extends inside the outer tubular portion (16) of the sheet (1), from the second holding member (2) to the distal tip (17), the inner portion (18) transiting into the outer portion (16) at the distal tip (17),
- a passage (19) defined inside the tubular inner portion (18) of the sheet (1), which passage (19) extends from the second holding member (2) and opens out at the distal tip (17),
in particular an instrument (50) as claimed in any of the preceding claims,
**characterized in that**
the instrument (50) further comprises at least one axially extendable stabilizing structure (20, 22; 11, 22) arranged inside the inner portion (18) of the sheet (1) in such a way that the inner portion (18) of the sheet (1) is prevented from closing the passage (19).

9. The instrument (50) as claimed in claim 8,
wherein the stabilizing structure (20, 22; 11, 22) comprises a plurality of stabilizing rings (11) arranged along the extension direction (D).

10. The instrument (50) as claimed in claim 8,
wherein the stabilizing structure (20, 22; 11, 22) comprises a helical spring (20) extending along the extension direction (D).

11. The instrument (50) as claimed in either of claims 9 and 10, wherein the stabilizing rings (11) or coils (21) of the helical spring (21), respectively, are connected to one another by at least one, preferably several controlling wires (22) extending in the extension direction (D).

12. The instrument (50) as claimed in claim 11,
wherein the controlling wires (22) extend through openings (29) provided in the stabilizing rings (11) or
coils (21) of the helical spring (20), respectively.

13. The instrument (50) as claimed in any of claims 8 to 12,
wherein the distal tip (17) comprises a tip head (4) which is attached to the stabilizing structure (20, 22; 11, 22) and arranged outside a chamber (25) that is at least partially enclosed by the inner portion (18) of the sheet (1) and the outer portion (16) of the sheet (1), wherein the tip head (4) further comprises a head opening (7) forming a distal end of the passage (19).

14. The instrument (50) as claimed in claimed in claim 13,
wherein the tip head (4) further comprises at least one measuring device (8, 9) for measuring a property of an inspected object, in particular at least one camera (8) and/or at least one ultrasound probe (9).

15. The instrument (50) as claimed in any of the preceding claims,
wherein the distal tip (17) of the instrument (50) comprises at least one guiding wheels (6) for guiding the sheet (1) at its transition from its inner portion (18) to its outer portion (16), wherein the at least one guiding wheel (6) is arranged inside a chamber (25) that is at least partially enclosed by the inner portion (18) of the sheet (1) and the outer portion (16) of the sheet (1).

16. The instrument (50) as claimed in claim 15,
wherein the distal tip (17) comprises at least two, in particular four, guiding wheels (6) which are rotatably arranged on a guiding ring (28) surrounding the passage (19).

17. A set, in particular a set (60), comprising an insertion instrument (50) as claimed in any of the preceding claims and at least one interior device (61) provided for extending through the passage (19) of the insertion instrument (50), wherein the interior device (61) is in particular selected from the group consisting of surgical, therapeutic or diagnostic instruments (61), sensors, robots, in particular autonomous robots or magnetically guided robots, cameras and drug delivery systems.
